# EUROPÄISCHE PATENTSCHRIFT

(11) **EP 0 592 453 B1**
(45) Veröffentlichungstag und Bekanntmachung des Hinweises auf die Patenterteilung: **16.12.1998**
(21) Anmeldenummer: 92908760.9
(22) Anmeldetag: 11.05.1992
(51) Int. Cl.: C07D 285/12, C07D 417/06, C07D 417/12, C07D 417/14, A61K 31/395

(54) **AMINOALKYLSUBSTITUIERTE 2-AMINO-1,3,4-THIADIAZOLE, IHRE HERSTELLUNG UND VERWENDUNG**
AMINOALKYL-SUBSTITUTED 2-AMINO-1,3,4-THIADIAZOLES, THEIR PREPARATION AND THEIR USE
2-AMINO-1,3,4-THIADIAZOLES A SUBSTITUANT AMINOALKYLE, LEUR PREPARATION ET LEUR UTILISATION

(30) Priorität: 15.06.1991 DE 4119755
(43) Veröffentlichungstag der Anmeldung: 20.04.1994
(73) Patentinhaber: BASF Aktiengesellschaft, 67063 Ludwigshafen (DE)
(72) Erfinder: RENDENBACH-MUELLER, Beatrice, D-6701 Waldsee (DE); UNGER, Liliane, D-6700 Ludwigshafen (DE); TESCHENDORF, Hans-Juergen, D-6724 Dudenhofen (DE)
(86) Internationale Anmeldenummer: EP9201028
(87) Internationale Veröffentlichungsnummer: WO9222541

(56) Entgegenhaltungen:
- GB-A- 1 053 085

## Beschreibung

Die Erfindung betrifft aminoalkylsubstituierte 2-Amino-1,3,4-thiadiazole, deren Herstellung sowie deren Verwendung zur Bekämpfung von Krankheiten.

In GB 1 053 085 werden Aminoalkylthiadiazole beschrieben, die antitussive, analgetische, antpyretische und hypoglycämische Wirkungen zeigen.

Es wurde nun gefunden, dap aminoalkylsubstituierte 2-Amino-1,3,4-thiadiazol-Derivate der Formel I 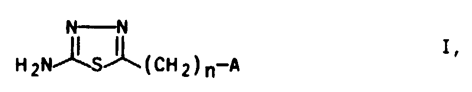 in der
n eine ganze Zahl von 2 bis 6 bedeutet und
A eine der Gruppen 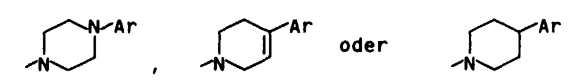 ist, wobei Ar für einen gegebenenfalls einfach durch C₁-C₅-Alkyl, C₁-C₅-Alkoxy, Halogen, Nitro, Hydroxy, Trifluormethyl oder Cyano substituierten Phenylring, einen Pyridyl-, Pyrimidinyl- oder Thienylrest steht, sowie deren Salze mit physiologisch verträglichen Säuren interessante pharmakologische Eigenschaften besitzen.
In der Formel I bedeutet A vorzugsweise 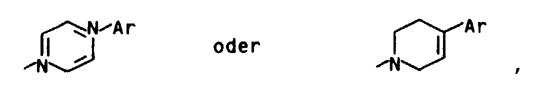 worin Ar unsubstitutiertes Phenyl, Pyridyl oder Pyrimidinyl bedeutet, und n vorzugsweise die Zahl 2, 3 oder 4.

Die Verbindungen der Formel I lassen sich herstellen, indem man
a) ein ω-X-Alkylthiadiazol der Formel II, 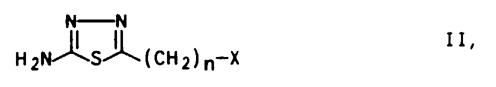 in der n wie eingangs definiert ist, und X für eine Abgangsgruppe wie Chlor, Brom oder RSO₂O- steht [R = C₁-C₄-Alkyl oder gegebenenfalls durch C₁-C₃-Alkyl oder Halogen substituiertes Phenyl], oder ein halogenwasserstoff-saures Salz dieser Verbindungen mit einem Amin der Formel III,

   HA III,

   in der A die angegebene Bedeutung hat, umsetzt oder
b) eine ω-Aminocarbonsäure der Formel IV,

   HO₂C-(CH₂)ₙ-A IV,

   in der A und n wie eingangs definiert sind, mit Thiosemicarbazid umsetzt
und die so erhaltenen Verbindungen gegebenenfalls in ihre Salze mit physiologisch verträglichen Säuren überführt.

Im Falle des Verfahrens a) erfolgen die Umsetzungen in der Schmelze, gewünschtenfalls auch in Gegenwart eines Lösungsmittels, z.B. Ethylacetat, Tetrahydrofuran, Dimethylformamid, Dimethoxyethan, Toluol oder Xylol, bei Temperaturen zwischen Raumtemperatur und dem Siedepunkt des verwendeten Lösungsmittels, vorzugsweise in Gegenwart einer Base wie Natriummethylat, Natriumethylat, Natriumhydrid, Natriumcarbonat, Kaliumcarbonat, eines Amins, z.B. Pyridin. Gegebenenfalls kann auch die Aminkomponente IV im überschuß als Reagenz, Base und Lösungsmittel fungieren.

Die als Ausgangsstoffe verwendeten ω-X-Alkylthiadiazole der Formel II können beispielsweise durch Umsetzung von ω-X-substituierten Carbonsäuren der Formel V,

HO₂C-(CH₂)ₙ-X V,

in der n und X die angegebenen Bedeutungen haben, mit Thiosemicarbazid hergestellt werden. Die Carbonsäuren der Formel V sind größtenteils literaturbekannt oder können nach bekannten Methoden hergestellt werden.

Die umsetzung nach Verfahren b) erfolgt in Gegenwart eines verdünnungs- oder Lösungsmittels, vorzugsweise einer starken Säure, beispielsweise konzentrierter Schwefelsäure, und zweckmäßigerweise bei Temperaturen zwischen Raumtemperatur und der Siedetemperatur des verwendeten Lösungsmittels. Die vollständige Umsetzung hängt von den Edukten ab und ist im allgemeinen innerhalb von 1 bis 6 Stunden beendet. Das Reaktionsprodukt kann in üblicher Weise isoliert und gereinigt werden. Die als Ausgangstoffe verwendeten ω-Aminocarbonsäuren der Formel IV sind zum Teil bekannt oder können in an sich bekannter Weise durch Umsetzung der ω-substituierten Carbonsäuren der Formel V mit Aminen der Formel III erhalten werden.

Die als Ausgangstoffe verwendeten ω-Aminocarbonsäuren der Formel IV sind literaturbekannt oder können durch Alkylierung der Amine der Formel III mit Carbonsäuren der Formel V erhalten werden.

Gegebenenfalls werden die erhaltenen erfindungsgemäpen Verbindungen in ihre Säureadditionsalze mit physiologisch verträglichen Säuren überführt. Als übliche physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere können aus Fortschritte der Arzneimittelforschung Bd. 10, S. 224 f., Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol oder Propanol, einem halogenierten Kohlenwasserstoff wie Methylenchlorid, einem Ether wie Methyl-t-butylether oder Diisopropylether, einem Keton wie Aceton oder Butanon oder einem Ester wie Essigsäureethylester erhalten. Zur besseren Kristallabscheidung können auch Mischungen der genannten Lösungsmittel verwendet werden. Darüberhinaus können pharmazeutisch vertretbare wäßrige Lösungen von Säureadditonsverbindungen der erfindungsgemäpen Verbindungen I durch Auflösen der freien Basen in einer wäßrigen Säurelösung hergestellt werden.

Die erfindungsgemäpen Verbindungen eignen sich zur Bekämpfung von Krankheiten, insbesondere zur Behandlung von Erkrankungen des zentralen Nervensystems (z.B. Parkinsonismus, Schizophrenie) sowie von erhöhtem Blutdruck. Sie weisen insbesondere wertvolle Eigenschaften als Dopaminagonisten, teilweise mit Selektivität für präsynaptische Dopaminrezeptoren, oder als Dopaminantagonisten auf. Die Verbindungen der Formel I zeigen Affinität zum Dopamin-Rezeptor in Rezeptorbindungstests; sie inhibieren die Motilität an Mäusen (gemessen in Lichtschrankenkäfigen) und beeinflussen das Rotationsverhalten an Ratten mit einseitigen 6-Hydroxydopamin-Läsionen der Substantia nigra (Brain Research 24, 485-493, 1970).

Die Wirkungen der neuen Verbindungen lassen sich im Rezeptorbindungstest zeigen:

Striata von Ratten (Sprague Dawley, Charles River) wurden sofort nach Entnahme in 0,32 M Saccharoselösung (0°C) homogenisiert. Das Homogenat wurde filtriert über Gaze, das Filtrat bei 1000 g zentrifugiert (5 min bei 4°C) und der resultierende überstand bei 40000 g zentrifugiert (10 min, 4°C). Der Rückstand (Membranen) wurde in Inkubationspuffer (50 mM Tris-HCl, 1 mM MgCl₂ und 0,1 % Ascorbinsäure, pH 7,4) aufgenommen und 20 min bei 37°C inkubiert. Anschliepend wurde durch Resuspension und Rezentrifugation 2x mit Inkubationspuffer gewaschen. Die Membranen wurden portionsweise in flüssigem Stickstoff eingefroren.

Die Testansätze (1 ml) setzten sich zusammen aus Membranen (380 µg Protein), 1 nM 3H-ADTN (NEN, Dreieich Germany, spez. Radioaktivität 1,4 TBq/mmol) und 0,1 µM SCH 23390 (totale Bindung) oder a) mit zusätzlich 50 nM Spiperon (unspezifische Bindung) oder b) mit Prüfsubstanz. Die Ansätze wurden als Triplikate hergestellt.

Nach beendeter Inkubation (40 min bei 25°C) wurden die Ansätze über Glasfaserfilter (Whatman GF/B) filtriert und kurz mit eiskaltem Waschpuffer (Tris-HCl, pH 7,4) gewaschen. Die auf den Filtern zurückgehaltene Radioaktivität wurde mittels Flüssigkeitszintillationsmessung bestimmt. Die unspezifische Bindung betrug ca. 40 - 50 % der totalen Bindung.

Die Auswertung der Kompetitionskurven und die Bestimmung der Dissoziationskonstante erfolgte über iterative nichtlineare Regressionsanalyse in Anlehnung an das Programm "Ligand" (Muson und Rodbard: Anal. Biochem. 107, 220, 1980).

Affinität der Prüfsubstanzen zum Dopamin-D₂-Rezeptor

| Beispiel | Ki (nM) |
|---|---|
| 4 | 30 |
| 7 | 30 |
| 10 | 32 |
| 18 | 5 |
| 19 | 10 |
| 21 | 44 |
| 24 | 37 |

Die erfindungsgemäpen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 500 mg pro Patient und Tag bei oraler Gabe und etwa 1 bis 100 mg pro Patient und Tag bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidatien und/oder Treibgasen verarbeitet werden (vgl. H. Sucker et al: Pharmazeutische Technologie, Thieme-Verlag, Stuttgart, 1978). Die so erhaltenen Applikationen enthalten den Wirkstoff normalerweise in einer Menge von 1 bis 99 Gew.-%.

Die folgenden Beispiele sollen die Erfindung näher erläutern:

### Beispiel 1

2-Amino-5-[2-(4-phenylpiperazinyl)-ethyl]-1,3,4-thiadiazol-dihydrochlorid
   4,5 g 2-Amino-5-(2-chlorethyl)-1,3,4-thiadiazol, 8,9 g 4-Phenylpiperazin und 0,2 g Natriumodid wurden in 70 ml Toluol 4 h auf 80°C erwärmt. Nach Zugabe von 3,8 ml Triethylamin wurde weitere 5 h bei dieser Temperatur gerührt, auf RT abgekühlt, der entstandene Feststoff abgesaugt und in Methylenchlorid/2N HCl verteilt. Die wäßrige Phase wurde abgetrennt und mit 5N NaOH alkalisch gestellt. Der bei pH 6 - 7 ausfallende Feststoff wurde abgesaugt, mit Wasser und Aceton gewaschen, in Ethanol aufgenommen und mit etherischer HCl versetzt. Das gebildete Kristallisat wurde abgesaugt und im Vakuum getrocknet.
   Ausbeute 2,5 g (25 %) Fp.: 186 - 188°C
   Analog Beispiel 1 wurden hergestellt:
2. 2-Amino-5-[2-(4-pyrimid-2-ylpiperazinyl)-ethyl]-1,3,4-thiadiazol-trihydrochlorid
   Ausbeute: 27 % Fp.: 148°C (Zersetzung)
3. 2-Amino-5-[2-(4-phenylpiperidinyl)-ethyl]-1,3,4-thiadiazoldihydrochlorid
   Ausbeute: 54 % Fp.: 178 - 181°C
4. 2-Amino-5-[2-(1,2,3,6-tetrahydro-4-phenylpyridyl)-ethyl]-1,3,4-thiadiazol-hydrochlorid
   Ausbeute: 79 % Fp.: 183 - 184°C
5. 2-Amino-5-[2-(4-pyrimidin-2-ylpiperazinyl)-ethyll-1,3,k-thiadiazol-hydrochlorid
   Ausbeute: 22 % Fp.: 140°C (Zersetzung)
6. 2-Amino-5-[2-(4-(3-methoxyphenyl)-piperazinyl)-ethyl]-1,3,4-thiadiazol-hydrochlorid
   Ausbeute: 31 % Fp.: 180°C (Zersetzung)
7. 2-Amino-5-[2-(4-(4-chlorphenyl)-1,2,3,6-tetrahydropyridyl)-ethyl]-1,3,4-thiadiazol-dihydrochlorid
   Ausbeute: 20 % Fp.: 154°C (Zersetzung)
8. 2-Amino-5-[2-(4-(2-fluorphenyl)-piperazinyl)-ethyl]-1,3,4-thiadiazol-hydrochlorid
   Ausbeute: 27 % Fp.: 187 - 189°C
9. 2-Amino-5-[3-(4-phenylpiperidinyl)-propyl]-1,3,4-thiadiazolhydrochlorid
   Ausbeute: 20 % Fp.: 225 - 226°C
10. 2-Amino-5-[3-(1,2,3,6-tetrahydro-4-phenylpyridinyl)-propyl]-1,3,4-thiadiazol-hydrochlorid
   Ausbeute: 17 % Fp.: 170 - 172°C
11. 2-Amino-5-[3-(4-(2-fluorphenyl)-piperazinyl)-propyl)-1,3,4-thiadiazol-hydrochlorid
   Ausbeute: 22 % Fp.: 165 - 166°C
12. 2-Amino-5-[3-(4-phenylpiperazinyl)-propyl]-1,3,4-thiadiazolhydrochlorid
   Ausbeute: 42 % Fp.: 199 - 201°C
13. 2-Amino-5-[3-(4-(2-nitrophenyl)-piperazinyl)-propyl]-1,3,4-thiadiazol-hydrochlorid
   Ausbeute: 19% Fp.: 154 - 155°C
14. 2-Amino-5-[4-(4-phenylpiperazinyl)-butyl)-1,3,4-thiadiazolhydrochlorid
   Ausbeute: 23 % Fp.: 244 - 245°C
15. 2-Amino-5-[2-(4-(2-nitrophenyl)-piperazinyl)-ethyl]-1,3,4-thiadiazol-dihydrochlorid
   Ausbeute: 23 % Fp.: 196°C
16. 2-Amino-5-[2-(4-(3-trifluormethylphenyl)-piperazinyl)-ethyl]-1,3,4-thiadiazol-trihydrochlorid
   Ausbeute: 17% Fp.: 180°C
17. 2-Amino-5-[2-(4-(2-cyanophenyl)-piperazinyl)-ethyl]-1,3,4-thiadiazol-dihydrochlorid
   Ausbeute: 24% Fp.: 181°C (Zersetzung)
18. 2-Amino-5-[4-(1,2,3,6-tetrahydro-4-phenylpyridinyl)-butyl]-1,3,4-thiadiazol
   Ausbeute: 22 % Fp.: 204 - 205°C
19. 2-Amino-5-[5-(1,2,3,6-tetrahydro-4-phenylpyrimidinyl)-pentyl]-1,3,4-thiadiazol-fumarat
   Ausbeute: 26 % Fp.: 163 - 165°C
20. 2-Amino-5-[3-(4-pyridin-2-ylpiperazinyl)-propyl]-1,3,4-thiadiazol
   Ausbeute: 43 % Fp.: 195 - 197°C
21. 2-Amino-5-[4-(4-pyridin-2-ylpiperazinyl)-butyl]-1,3,4-thiadiazol
   Ausbeute: 32 % Fp.: 170 - 171°C
22. 2-Amino-5-[3-(4-pyrimidin-2-ylpiperazinyl)-propyl]-1,3,4-thiadiazol
   Ausbeute: 33 % Fp.: 183 - 184°C
23. 2-Amino-5-[4-(4-pyrimidin-2-ylpiperazinyl)-butyl-1,3,4-thiadiazol
   Ausbeute: 25 % Fp.: 192°C
24. 2-Amino-5-[4-phenylpiperidinyl)-ethyl]-1,3,4-thiadiazol
   Ausbeute: 36 % Fp.: 187 - 188°C

Synthese der Ausgangsstoffe der Formel II
a. 2-Amino-5-(2-chlorethyl)-1,3,4-thiadiazol:
   Zu 65 g β-Chlorpropionsäure in 240 ml konz. Schwefelsäure wurden 65 g Thiosemicarbazid portionsweise zugegeben, wobei die Temperatur der Reaktionsmischung auf 70 - 80°C anstieg. Anschliepend wurde 1 h unter Rückflup gekocht, abgekühlt und auf Eiswasser (500 ml) gegossen. Mit Ammoniak wurde neutralisiert. Das ausgefallene Produkt wurde abgesaugt, mit Wasser gewaschen und im Vakuum getrocknet.
   Ausbeute: 56 g (57 %) Fp.: 133 - 137°C
   Analog wurden hergestellt:
b. 2-Amino-5-(3-chlorpropyl)-1,3,4-thiadiazol Fp.: 142 - 145°C
c. 2-Amino-5-(4-chlorbutyl)-1,3,4-thiadiazol Fp.: 204 - 205°C
d. 2-Amino-5-(5-brompentyl)-1,3,4-thiadiazol Fp.: 118 - 120°C

### Beispiele für galenische Applikationsformen:

A) Auf einer Tablettenpresse werden in üblicher Weise Tabletten folgender Zusammensetzung gepreβt:

| | |
|---|---|
| 40 mg | Substanz des Beispiels 1 |
| 120 mg | Maisstärke |
| 13,5 mg | Gelatine |
| 45 mg | Milchzucker |
| 2,25 mg | Aerosile (chemisch reine Kieselsäure in |
| | submikroskopisch feiner Verteilung) |
| 6,75 mg | Kartoffelstärke (als 6 %iger Kleister) |

B)

| | |
|---|---|
| 20 mg | Substanz des Beispiels 4 |
| 60 mg | Kernmasse |
| 60 mg | Verzuckerungsmasse |

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Kalk. Die so hergestellten Dragees werden anschliepend mit einem magensaftresistenten überzug versehen.
C) 10 g Substanz des Beispiels 2 werden in 5000 ml Wasser unter Zusatz von NaCl gelöst und mit 0,1N NaOH auf pH 6,0 eingestellt, so dap eine blutisotonische Lösung entsteht. Jeweils 1 ml dieser Lösung wird in Ampullen gefüllt und sterilisiert.

## Patentansprüche

1. Aminoalkylsubstituierte 2-Amino-1,3,4-thiadiazol-Derivate der Formel I, 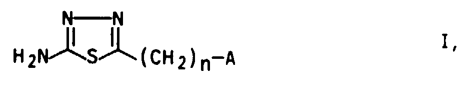 in der
n eine ganze Zahl von 2 bis 6 bedeutet und
A eine der Gruppen 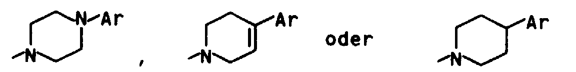 ist, wobei Ar für einen gegebenenfalls einfach durch C₁-C₅-Alkyl, C₁-C₅-Alkoxy, Halogen, Nitro, Hydroxy, Trifluormethyl oder Cyano substituierten Phenylring, einen Pyridyl-, Pyrimidinyl- oder Thienylrest steht sowie deren Salze mit physiologisch verträglichen Säuren.

2. Verfahren zur Herstellung der aminoalkylsubstituierten 2-Amino-1,3,4-thiadiazol-Derivate gemäp Anspruch 1, dadurch gekennzeichnet, daß man
a) ein ω-X-Alkylthiadiazol der Formel II, 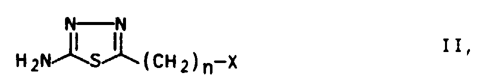 in der n wie eingangs definiert ist, und X für eine Abgangsgruppe wie Chlor, Brom oder RSO₂O- steht [R = C₁-C₄-Alkyl oder gegebenenfalls durch C₁-C₃-Alkyl oder Halogen substituiertes Phenyl] oder ein halogenwasserstoff-saures Salz dieser Verbindung mit einem Amin der Formel III,
HA III
in der A die angegebene Bedeutung hat, umsetzt oder
b) eine ω-Aminocarbonsäure der Formel IV,
HO₂C-(CH₂)ₙ-A IV,
in der A und n wie eingangs definiert sind, mit Thiosemicarbazid umsetzt
und die so erhaltenen Verbindungen gegebenenfalls in ihre Salze mit physiologisch verträglichen Säuren überführt.

3. Aminoalkylsubstituierte 2-Amino-1,3,4-thiadiazol-Derivate der Formel I gemäp Anspruch 1 zur Verwendung bei der Bekämpfung von Krankheiten.

4. Verwendung eines aminoalkylsubstituierten 2-Amino-1,3,4-thiadiazol-Derivates der Formel I nach Anspruch 1 zur Herstellung eines Arzneimittels gegen Erkrankungen des zentralen Nervensystems oder Erkrankungen, die mit erhöhtem Blutdruck einhergehen.

## Claims

1. An aminoalkyl-substituted 2-amino-1,3,4-thiadiazole derivative of the formula I 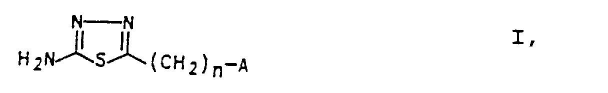 where
n is an integer from 2 to 6, and
A is 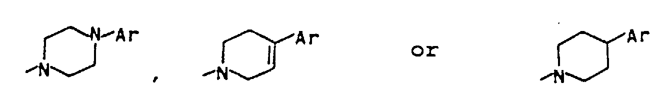 where Ar is phenyl which is unsubstituted or monosubstituted by C₁-C₅-alkyl, C₁-C₅-alkoxy, halogen, nitro, hydroxyl, trifluoromethyl or cyano, or is pyridyl, pyrimidinyl or thienyl, and the salts thereof with physiologically tolerated acids.

2. A process for preparing the aminoalkyl-substituted 2-amino-1,3,4-thiadiazole derivatives as claimed in claim 1, which comprises
a) reacting an ω-X-alkylthiadiazole of the formula II 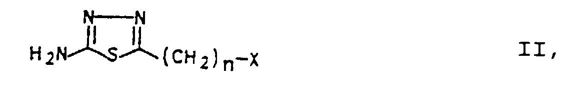 where n is as defined above, and X is a leaving group such as chlorine, bromine or RSO₂O- [R = C₁-C₄-alkyl or phenyl which is unsubstituted or substituted by C₁-C₃-alkyl or halogen], or a hydrohalide of this compound, with an amine of the formula III
HA III,
where A has the stated meanings, or
b) reacting an ω-amino carboxylic acid of the formula IV
HO₂C-(CH₂)ₙ-A IV,
where A and n are as defined above, with thiosemicarbazide,
and, where appropriate, converting the resulting compounds into their salts with physiologically tolerated acids.

3. An aminoalkyl-substituted 2-amino-1,3,4-thiadiazole derivative of the formula I as claimed in claim 1 for use for controlling diseases.

4. The use of an aminoalkyl-substituted 2-amino-1,3,4-thiadiazole derivative of the formula I as claimed in claim 1 for producing a drug for disorders of the central nervous system or disorders associated with elevated blood pressure.

## Revendications

1. Dérivés du 2-amino-1,3,4-thiadiazole à substituant aminoalkyle répondant à la formule I 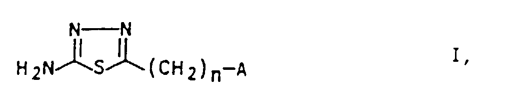 dans laquelle
n est un nombre entier allant de 2 à 6 et
A représente l'un des groupes 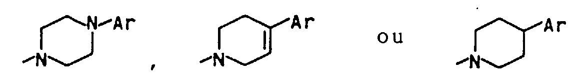 dans lesquels Ar représente un cycle phényle portant éventuellement un substituant alkyle en C1-C5, alcoxy en C1-C5, halogéno, nitro, hydroxy, trifluorométhyle ou cyano, un radical pyridyle, pyrimidinyle ou thiényle, et leurs sels d'acides acceptables pour l'usage pharmaceutique.

2. Procédé de préparation des dérivés du 2-amino-1,3,4-thiadiazole à substituant aminoalkyle selon la revendication 1, caractérisé par le fait que
a) on fait réagir un oméga-X-alkylthiadiazole de formule II 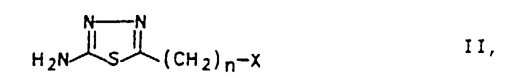 dans laquelle n a les significations indiquées ci-dessus et X représente un substituant éliminable tel que le chlore, le brome ou un groupe RSO₂O- (R = alkyle en C1-C4 ou phényle éventuellement substitué par alkyle en C1-C3 ou halogène) ou un halogénhydrate de ce composé, avec une amine de formule III
HA III,
dans laquelle A a les significations indiquées ci-dessus, ou bien
b) on fait réagir un acide oméga-aminocarboxylique de formule IV
HO₂C-(CH₂)ₙ-A IV,
dans laquelle A et n ont les significations indiquées ci-dessus, avec le thiosemicarbazide, et le cas échéant on convertit les composés ainsi obtenus en leurs sels d'acides acceptables pour l'usage pharmaceutique.

3. Dérivés du 2-amino-1,3,4-thiadiazole à substituant aminoalkyle de formule I de la revendication 1 pour l'utilisation dans le traitement de maladies.

4. Utilisation d'un dérivé du 2-amino-1,3,4-thiadiazole à substituant aminoalkyle de formule I de la revendication 1 pour la préparation d'un médicament servant au traitement des maladies du système nerveux central ou des maladies liées à l'hypertension.
